# EUROPEAN PATENT APPLICATION

(11) **EP 2 036 506 A1**
(43) Date of publication of application: **18.03.2009**
(21) Application number: 08102415.0
(22) Date of filing: 07.03.2008
(51) Int. Cl.: A61B 17/34, A61F 9/007

(54) **Trocar cannula**

(30) Priority: 17.09.2007 US 856382
(71) Applicant: Alcon, Inc., 6331 Hünenberg (CH)
(72) Inventor: Attinger, Jürg, 8260, Stein am Rhein (CH)
(74) Representative: Hanna, Peter William Derek

(57) **Abstract**

A cannula (10) is provided having a tube (11) that is longitudinally split and open along the entire, or along substantially the entire, length of the tubing, so as to provide an open channel (20). Attached to the proximal end of the tubing is a hub (12) that is also longitudinally split and open. The hub acts as a stop to prevent the entire cannula from entering a surgical incision. The cannula is suitable for ophthalmic microsurgery and permits curved instruments to be introduced through a very small incision in the sclera.

## Description

This invention relates to ophthalmic surgical equipment and more particularly to posterior segment ophthalmic surgical equipment.

### Background of the Invention

Microsurgical instruments typically are used by surgeons for any manipulations or removal of tissue from delicate and restricted spaces in the human body, particularly in surgery on the eye, and more particularly in procedures for manipulations or removal of the vitreous body, blood, scar tissue, or the crystalline lens. Such instruments may be hand-held, but often include a control console and a surgical handpiece with which the surgeon dissects, manipulates and/or removes the tissue. The handpiece has a surgical tool such as a vitreous cutter probe or an ultrasonic fragmentor for cutting or fragmenting the tissue and is connected to the control console by a long air pressure (pneumatic) line or power cable and by long conduits, cable, optical cable or flexible tubes for supplying an infusion fluid to the surgical site and for withdrawing or aspirating fluid and cut/fragmented tissue from the site. The cutting, infusion and aspiration functions of the handpiece are controlled by the remote control console that not only provides power for the surgical handpiece(s) (e.g., a reciprocating or rotating cutting blade or an ultrasonically vibrated needle), but also controls the flow of infusion fluid and provides a source of reduced pressure (relative to atmosphere) for the aspiration of fluid and cut/fragmented tissue. The functions of the console are controlled manually by the surgeon, usually by means of a foot-operated switch or proportional control.

During posterior segment surgery, the surgeon typically uses several instruments during the procedure. This requires that these instruments be inserted into, and removed out of the incision. This repeated removal and insertion can cause trauma to the eye at the incision site. To address this concern, cannulae were developed at least by the mid1980s. These devices consist of a narrow tube with an attached hub. An incision is made, and the tube is inserted into the incision up to the hub, which acts as a stop, preventing the tube from entering the eye completely. Surgical instruments can be inserted into the eye through the tube, and the tube protects the incision from repeated contact by the instruments. In addition, the surgeon can use the instrument, by manipulating the instrument when the instrument is inserted into the eye through the tube, to help position the eye during surgery. Prior art cannulae are necessarily small in diameter. Small diameter cannulae allow the wound created in the eye to be self-sealing, and not requiring any sutures and the small incision size reduces overall trauma to the eye, providing for faster recovery after surgery. While surgical instruments having straight or relatively straight shafts pass easily into and out of the internal bore of these prior-art cannulae, many surgical instruments are curved in order to access portions of the posterior chamber of the eye located more anteriorly. If the shafts of these curved instruments have any significant bends, the extremely small bore diameter of prior art cannulae will prevent the instrument from passing through the cannulae.

Accordingly, a need continues to exist for a relatively small bore cannula that allows curved instruments to be inserted into a surgical site.

### Brief Summary of the Invention

The present invention improves upon prior art by providing a cannula having a tube that is longitudinally split and open along the entire, or along substantially the entire, length of the tubing, in accordance with claims which follow. Attached to the proximal end of the tubing is a hub that is also longitudinally split and open. The hub acts as a stop to prevent the entire cannula from entering the incision.

Accordingly, an objective of the present invention to provide an ophthalmic cannula.

Another objective of the present invention to provide an ophthalmic cannula having a tube that is longitudinally split and open along the entire, or along substantially the entire, length of the tubing.

A further objective of the present invention to provide an ophthalmic cannula that permits curved instruments to be inserted into the surgical site.

Other objectives, features and advantages of the present invention will become apparent with reference to the drawings, and the following description of the drawings and claims.

### Brief Description of the Drawings

FIG. 1 is an enlarged perspective view of a prior art cannula.
FIG. 2 is an enlarged perspective view of the cannula of the present invention.
FIG. 3 is an enlarged perspective view of a first alternative embodiment of the cannula of the present invention.
FIG. 4 is an enlarged cross-sectional view of the cannula of the present invention taken at line 4-4 in FIG. 1.
FIG. 5 is an enlarged perspective view of a second alternative embodiment the cannula of the present invention.

### Detailed Description of the Preferred Embodiments

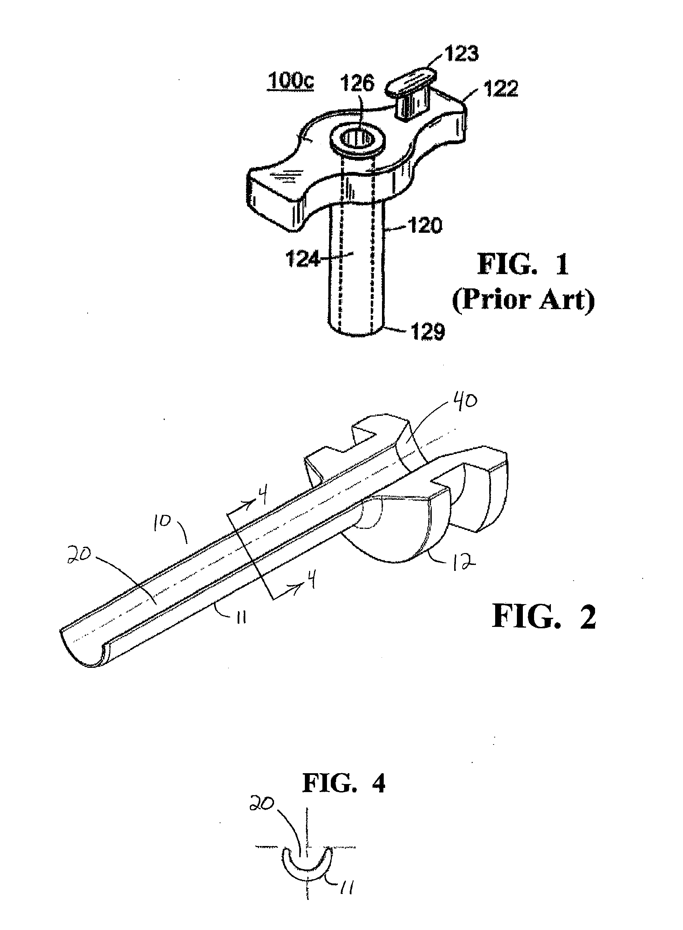

As best seen in FIG. 1, prior art cannula 100c includes tube 120 and hub 122 that is affixed to tube 120. Tube 120 includes lumen 124 extending the entire length of cannula 100c between hub 122 and distal end 129 of tube 120. Tube 120 contains proximal aperture 126 that communicates with lumen 124. Hub 122 also includes feature 123 that is configured so as to provide a mechanism by which the surgeon can grasp cannula 100c for insertion into and removal from the eye. Tube 120 is of sufficient length to extend through the sclera and enter the posterior chamber of an eye.

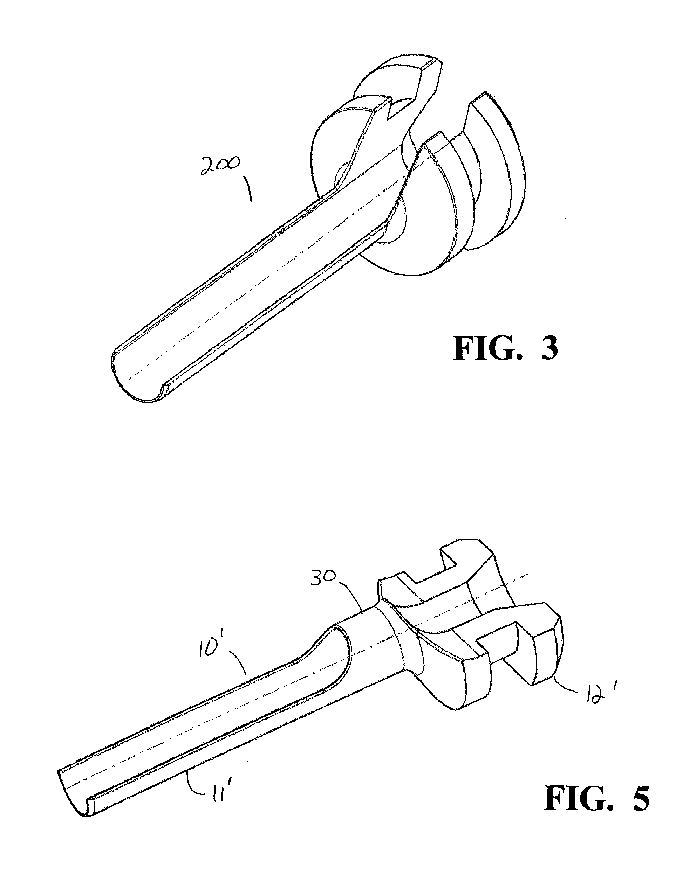

As seen in FIG. 2, cannula 10 of the present invention consists of tube 11 and enlarged hub 12. Tube 11 is of sufficient length to extend through the sclera and enter the posterior chamber. Tube 11 and hub 12 are made from any suitable material such as metal or thermoplastic. The internal dimensions of tube 11 may be of any suitable size, such as 20, 23 or 25 gauge or other. Tube 11 may contain a rib (not shown) to help prevent cannula 10 from becoming dislodged from sclera. Hub 12 has central aperture 40 which, as best seen in FIG. 2, may be generally funnel shaped so as to assist in the entry of surgical tools into aperture 40 and tube 11. As best seen in FIGS. 2-4, tube 11 and hub 12 are longitudinally split along the entire length of tube 11 so as to form open channel 20. While tube 11 and hub 12 are illustrated in FIG. 2 as being split roughly in half, other proportions may also be used, as shown by cannula 200 in FIG. 3, which includes a groove formed therein. Such a construction allows cannula 10 to act as a guide for the insertion of surgical instruments through the sclera and into the posterior chamber without restricting the shape of the shaft of the surgical instruments.

Alternatively, as seen in FIG. 5, tube 11' of cannula 10' may be only partially longitudinally split, leaving full portion 30 of tube 11' where tube 11' enters the sclera fully encased. Such a construction helps to reinforce tube 11' at the sclera adjacent to hub 12', thus helping to keep the conjunctiva (not shown) aligned with the sclera, acts as a pivot point for the surgical instrument during use and also helps protect the incision site from trauma caused by repeated insertions and removals of surgical instruments. While certain embodiments of the present invention have been described above, these descriptions are given for purposes of illustration and explanation. Variations, changes, modifications and departures from the systems and methods disclosed above may be adopted without departure from the scope of the present invention.

## Claims

1. A cannula (10, 10', 200), comprising:
a) a tube (11,11') having a distal end and a proximal end; and
b) a hub (12,12') connected to the tube at the proximal end of the tube, **characterized in that** the tube and the hub are longitudinally split along substantially the entire length of the tube and the hub so as to form an open channel (20).

2. The cannula (10') of claim 1, wherein the tube (11') is split longitudinally along a partial length of the tube thereby leaving a portion (30) of the tube unsplit, and wherein the hub (12') is connected to the tube at the proximal end of the tube adjacent the unsplit portion of the tube, the hub (12') being split longitudinally along its entire length.

3. The cannula (10,10') of claim 1 or claim 2, wherein the tube (11,11') and the hub (12,12') are longitudinally split, roughly in half.

4. The cannula (200) of claim 1, wherein the tube (11) is longitudinally split, roughly in half, and wherein the hub (12) is longitudinally split by means of a groove formed therein.
